(19) Europäisches Patentamt

European Patent Office

Office européen des brevets

(11) **EP 1 288 264 A1**

(12) **EUROPEAN PATENT APPLICATION**
published in accordance with Art. 158(3) EPC

(43) Date of publication:
05.03.2003 Bulletin 2003/10

(51) Int Cl.⁷: **C08L 101/00**, C08K 5/00,
A61L 31/04, A61L 24/06,
A61K 9/08, A61P 17/02,
A61P 7/04, A61P 43/00

(21) Application number: 01932200.7

(22) Date of filing: 22.05.2001

(86) International application number:
PCT/JP01/04277

(87) International publication number:
WO 01/090252 (29.11.2001 Gazette 2001/48)

(84) Designated Contracting States:
AT BE CH CY DE DK ES FI FR GB GR IE IT LI LU
MC NL PT SE TR
Designated Extension States:
AL LT LV MK RO SI

(30) Priority: 22.05.2000 JP 2000154410

(71) Applicant: **Mori, Yuichi**
Yokohama-shi, Kanagawa 236-0045 (JP)

(72) Inventor: **Mori, Yuichi**
Yokohama-shi, Kanagawa 236-0045 (JP)

(74) Representative: **Patentanwälte**
**Ruff, Wilhelm, Beier, Dauster & Partner**
**Kronenstrasse 30**
**70174 Stuttgart (DE)**

(54) **GELLING COMPOSITION**

(57) A composition having a gel-forming property, which comprises at least a polymer component, and a gel-forming agent for converting the polymer component into a gel state; and shows at least two kinds of the gel formations including a relatively fast gel formation F and a relatively slow gel formation S. The difference be- tween the time (Tf) of the gel formation F and the time (Ts) of the gel formation S may preferably be about 30 minutes or more. Based on the above constitution, it is possible to realize a good balance between the short-term adhesion and long-term adhesion.

**EP 1 288 264 A1**

**Description**

Technical Field

The present invention relates to a composition having a gel-forming property which exhibits two kinds of gel-forming characteristics which have never been disclosed in the prior art. More specifically, the present invention relates to a composition having a gel-forming property, which comprises at least a polymer component, and a gel-forming agent capable of forming the polymer component into a gel, which shows at least two kinds of gel-forming characteristics including a relatively fast gel formation F and a relatively slow gel formation S, wherein these gel formations satisfy a predetermined relationship. For example, the gel-forming composition according to the present invention may preferably be achieved by combining gel-forming components showing mutually different gel-forming mechanisms with each other (e.g., a gel-forming component F providing a reversible gel formation, and a gel-forming component S providing an irreversible gel formation).

Background Art

The gel-forming composition according to the present invention may be applied, without particular limitation, to the fields which require a good balance between a short-term adhesion (or adhesive force), and a long-term adhesion. In this specification, however, there will be first described a background art relating to a composition having a gel-forming property (particularly, a composition having a hydrogel-forming property) according to one aspect of the present invention wherein a physical gel formation and a chemical gel formation are combined with each other.

Hydrogels which have heretofore been developed are classified into two kinds thereof depending on the kinds of crosslinking bonds, as a basis constituting the gels. That is, they are classified into chemical gels wherein the crosslinking is predominantly formed by covalent bonds; and physical gels wherein the crosslinking is predominantly formed by a physical interaction such as hydrogen bonding, electrostatic bonding, hydrophobic bonding, and van der Waals force.

The above-mentioned physical energies are generally much smaller than the covalent bond energy, and therefore many of physical gels have a tendency that they readily cause a phase transition between the sol state and the gel state depending on a change in the temperature or concentration. On the other hand, in case of the chemical gel, the crosslinking energy due to the covalent bonding is large and, therefore, in general, this type of gel is very stable, and has a tendency not to transition into a sol state due to a change in the temperature or concentration.

Among the physical gels, examples of a certain type thereof (i.e., thermo-reversible hydrogel) which causes a transition between the gel state and the sol state due to a temperature change, may include, e.g., hydrogels such as those utilizing gelatine or agar, which cause a transition from the gel state into the sol state due to heating. On the other hand, contrary to such type of hydrogels, there are present some hydrogels which are converted from a sol state into a gel state due to heating. The crosslinking of the former type of thermo-reversible hydrogels is usually constituted by hydrogen bonding, electrostatic bonding, van der Waals force, microcrystal formation force, etc. The bonding force of this type is decreased as the temperature is increased, and therefore these gels are converted from a gel state into a sol state due to heating.

On the other hand, the crosslinking of the latter type of thermo-reversible hydrogel is usually constituted by hydrophobic bonding, and this type of hydrophobic bonding force is increased when the temperature increases, and therefore this gel is converted from a sol state into a gel state due to heating. As examples of such a thermo-reversible hydrogel, there have been developed thermo-reversible hydrogels comprising a polymer chain having a cloud point such as poly-N-isopropylacrylamide (PNIPAAm) or polypropylene oxide (PPO); and a hydrophilic polymer chain such as polyethylene oxide (PEO) which has been combined with the polymer chain having a cloud point in the form of block-type or graft-type (for example, JP-A (KOKAI; Japanese Unexamined Patent Publication) No. 4-16114, JP-A No. 5-18697, JP-A No. 7-187902, and JP-A No. 7-187903 may be refereed to).

Herein, the above polymer chain having a cloud point is water-soluble at a temperature lower than the cloud point, but is changed into a hydrophobic state (water-insoluble) at a temperature higher than the cloud point. Accordingly, such a polymer chain forms a crosslinking structure at a temperature higher than the cloud point due to the hydrophobic bonding between polymer chains so as to be converted into a gel state. On the other hand, the above-mentioned hydrophobic bonding between polymer chains is weakened at a temperature lower than the cloud point, and therefore such a polymer chain shows a phenomenon (so-called thermo-gelling type thermo-reversible hydrogel formation) that the polymer chains assume a sol state due to breakage of the above crosslinking structure.

Among the above-mentioned gels, in the case of chemical gels having a crosslinking structure comprising covalent bonding, the stability of the gel state is generally excellent but, usually, it is very difficult to effect a conversion between the sol state and the gel state by utilizing ambient conditions (e.g., under a physiological condition; 37-39 °C, pH 7.3 to 7.6). Accordingly, when a chemical gel is intended to be used for an application or usage wherein the gel-forming material is converted into a gel after the elapse of a certain period (for example, "in vivo adhesive" to be used in a living body in a medical field), it is necessary that the gel-forming material in a sol state is administered into a living body before such a material forms a crosslinking structure comprising covalent bonding, and then the material is caused to produce a chemical reaction in the living body so as to be converted into a gel state, to thereby position the thus formed gel at the injection site of the living body.

However, when the conventional chemical gels are used for such an application (in the case of the medical field, for example, intravascular embolus-forming agent, hemostatic agent, in-vivo adhesive, adhesion-preventing agent, wound-covering agent, base material for drug delivery, etc.) wherein a gel is intended to be formed after the elapse of a certain period, there have been posed various problems such that in some cases, the rate of the gel-forming chemical reaction is too fast and the gel-forming material is converted into a gel state in the course of the administration of the gel-forming material into a living body, and therefore the administration of the gel-forming material per se becomes difficult; or in some cases, the rate of the gel-forming chemical reaction is too slow and it becomes difficult to stably position the resultant gel at the intended site of the living body where the gel-forming material is administered into the living body; etc.

Disclosure of Invention

An object of the present invention is to provide a gel-forming composition which can solve the above-mentioned problems encountered in the prior art, and can realize a good balance between the short-term adhesion and the long-term adhesion.

Another object of the present invention is to provide a gel forming composition which can easily be applied to a predetermined site (e.g., application thereof into a living body, etc., in the co-presence of a fluid) in a sol state thereof, can quickly be converted into a gel state, and further can stably be positioned or indwelled at an intended or predetermined site for a long period.

As a result of earnest study, the present inventor has found that it is extremely effective for solving the above object to use a combination of gel-forming components wherein the presence of the crosslinking structure based on a fast gel formation does not substantially hinder (or inhibit) a slow gel formation.

The composition having a gel-forming property is based the above discovery, and comprises: at least, a polymer component, and a gel-forming agent for converting the polymer component into a gel state; the gel-forming composition showing at least two kinds of the gel formations including a relatively fast gel formation and a relatively slow gel formation.

The present invention also provides a composition having a gel-forming property, comprising, at least, a polymer component, and a gel-forming agent for converting the polymer component into a gel state; the gel-forming composition showing at least two kinds of the gel formations including a relatively fast gel formation F and a relatively slow gel formation S, wherein the gel formation F and the gel formation S are not substantially hindered by each other.

The present invention further provides a composition having a gel-forming property, comprising, at least, a polymer component, and a gel-forming agent for converting the polymer component into a gel state; the gel-forming composition showing at least two kinds of the gel formations including a relatively fast gel formation F and a relatively slow gel formation S, wherein the gel formation F and the gel formation S have mutually different gel-forming mechanisms from each other.

The present invention further provides a composition having a gel-forming property, comprising, at least, a polymer component, and a gel-forming agent for converting the polymer component into a gel state; the gel-forming composition showing at least two kinds of the gel formations including a reversible and relatively fast gel formation F, and an irreversible and relatively slow gel formation S.

In the gel-forming composition according to the present invention having the above-mentioned constitution, the presence of the crosslinking structure based on the gel-forming component F for providing the relatively fast gel formation does not substantially hinder the gel formation S for providing the relatively slow gel formation; and the gel-forming component S does not substantially hinder the gel formation F. Accordingly, in the present invention, it is easy to realize a good balance between the short-term and instantaneous adhesion due to the rapid gel formation based on the gel-forming component F; and the sustained (or prolonged) and stable adhesion due to the delayed gel formation based on the gel-forming component S.

According to the present inventor's investigations and knowledge, the reason for a phenomenon that one of the above-mentioned gel formations does not substantially hinder the other of the above-mentioned gel formations in the gel-forming composition according to the present invention may be presumed as follows. That is, it is presumed that the gel-forming component S can substantially maintain a "liquid state" thereof during an appropriate period in the crosslinking structure formed by the "fast gel formation" of the gel-forming component F (or in the minute spaces in the individual "cells" constituting the crosslinking structure), and therefore the gel-forming component S can be converted into a gel state due to the "slow gel formation" even in the crosslinking structure of the gel-forming component F, and/or at the time of the "fast gel formation" of the gel-forming component F, the co-present gel-forming component S substantially behaves as a liquid or fluid, and therefore the "fast gel formation" of the gel-forming component F is not substantially affected by the presence of the slow gel-forming component S.

Further, according to the present inventor's investigations and knowledge, it is presumed that, at the time of the "slow gel formation" of the gel-forming component S, the presence of the crosslinking structure based on the fast gel-forming component F rather protects the gel formation S from the external environment (e.g., contact of the gel-forming component S with a fluid which can possibly hinder the "slow gel formation" of the gel-forming component S) so as to prevent the flowing-out, deficiency, deterioration, etc., of the gel-forming component S, and as a result, the crosslinking structure based on the fast gel-forming component F has a function of promoting the favorable gel formation of the gel-forming component S.

On the contrary, when the conventional physical gel or chemical gel is used singly, it is impossible to obtain the above-mentioned effect.

For example, when the above-mentioned thermo-gelling type thermo-reversible hydrogel is used, such a gel-forming material assumes a sol state at a lower temperature and assumes a gel state at a higher temperature, and therefore it is possible to embed a biological (or living body-related) substance (such as proteins, cells, and tissues/organs) in the gel substantially without imparting a damage to the biological substance, although such a biological substance can easily be damaged by heating. Further, it is also possible to easily recover the biological substance from the interior of the gel substantially without imparting damage to the biological substance, by subjecting the biological substance embedded in the gel to a lower temperature. In addition, it is possible to cause the gel to rapidly make a transition between the sol state and the gel state, simply by providing a temperature change within a physiologically acceptable condition in such a gel under physiological conditions such as pH, and concentration and kind of salt.

However, according to the present inventor's investigations, a phenomenon has been found that, when the thermo-gelling type thermo-reversible hydrogel is used at a site which is in frequent contact with a fluid (e.g., under physiological conditions such as those in the interior of a living body which is in frequent contact with body fluids), the resultant gel state formed by the body temperature is very unstable to a change such as those in temperature and concentration, and the gel is easily solubilized. According to the present inventor's investigation and knowledge, it is presumed that the bond energy of the hydrophobic bonding constituting the crosslinking structure is several Kcal/mol and is much smaller than the energy of the electrostatic bonding, microcrystal formation, covalent bonding, etc. According to the present inventor's experiments, it has been found that when the above-mentioned thermo-gelling type thermo-reversible hydrogel is used for the usage according to the present invention, such as intravascular embolus-forming agent, hemostatic agent, in-vivo adhesive, adhesion-preventing agent, wound-covering agent, and base material for drug delivery, such a gel can be administered into a living body in a sol state at a temperature lower than the body temperature, and can promptly be converted into a gel state at body temperature, to thereby position the gel at the injection site in the living body, but the stability of the gel in the living body is not good, as described hereinabove.

On the other hand, in the case of the chemical gel having the crosslinking structure comprising covalent bonding, the stability of the gel state thereof is excellent but, according to the present inventor's experiments, it has been found very difficult for the chemical gel to cause a transition between the sol and gel states at a site which is in frequent contact with a body fluid (e.g., under the physiological conditions such as those in the interior of a living body under which the gel is in contact with the body fluid).

According to the present inventor's experiments, it has been found that when the above-mentioned simple chemical gel having a crosslinking structure comprising covalent bonding is used, and the corresponding gel material is administered into a living body in the form of a sol state prior to the formation of the crosslinking structure therein, so that the gel material is intended to cause a chemical reaction to form a gel in the living body and to position the gel at the injection site, the rate of the gel-forming chemical reaction is too fast and the gel-forming material is converted into a gel state in the course of the administration of the gel-forming material into a living body, and therefore the administration of the gel-forming material per se becomes difficult; or the rate of the gel-forming chemical reaction is too slow and it becomes difficult to stably position the resultant gel at the intended site of the living body where the gel-forming material is administered into the living body.

Best Mode for Carrying Out the Invention

Hereinbelow, the present invention will be described in detail with reference to the accompanying drawings as desired. In the following description, "%" and "part(s)" representing a quantitative proportion or ratio are those based on mass, unless otherwise noted specifically.

(Combination of polymer and gel formation)

As long as the gel-forming composition according to the present invention comprises at least a polymer component and a gel-forming agent for converting the polymer component into a gel state, and the gel-forming composition shows at least two kinds of gel formations including the above-mentioned "fast gel formation" and "slow gel formation", the number of the polymer component and gel-forming agent constituting the gel-forming composition are not particularly limited and the mechanism of the two kinds of gel-forming composition is not particularly limited, either. In the present invention, it preferred to use, e.g., the following combinations as a combination of the above-mentioned component and gel formation.

The gel-forming composition according to the present invention shows at least two kinds of gel formations including a relatively fast gel formation F and a relatively slow gel formation S. The difference between the gel-forming time of the gel formation F (Tf) and the gel-forming time of the gel formation S (Ts) may preferably be about 30 minutes or more, more preferably two hours or more (particularly, six hours or more).

<Preferred combinations of polymer component and gel-forming agent>

|  | Number of the polymer component | Number of gel-forming agent |
|---|---|---|
| (1) | 1 | 1 |
| (2) | 1 | 2 |
| (3) | 2 | 1 |
| (4) | 2 | 2 |

Among the above embodiments, e.g., in the above embodiment (1), it is possible to achieve the "fast gel formation" by utilizing the physical gel formation of the above-mentioned one kind of a polymer component, and to achieve the "slow gel formation" by the chemical gel formation of the same polymer component.

Further, in the above embodiment (2), for example, it is possible to achieve the "fast gel formation" by the first chemical gel formation of the above one kind of a polymer component, and to achieve the "slow gel formation" by the second chemical gel formation of the same polymer component (however, the functional group relating to the gel formation is different from that relating to the first chemical gel formation).

In the above embodiment (3), for example, it is possible to achieve the "fast gel formation" by the chemical gel formation of the polymer component F, and to achieve the "slow gel formation" by the chemical gel formation of other polymer component S (for example, the functional group of the polymer component S is different from that of the polymer component F).

Furthermore, in the above embodiment (4), for example, it is possible to achieve the "fast gel formation" by a combination of the polymer component F and a gel-forming agent F, and to achieve the "slow gel formation" by a combination of other polymer component S (for example, the functional group of the polymer component S is different from that of the polymer component F) and a gel-forming agent S.

The above components, such as the polymer component and gel-forming agent constituting the composition according to the present invention, may be dissolved or dispersed in various kinds of solvents, as desired. The solvent to be used for such a purpose is not particularly limited. In an embodiment wherein the composition is to be applied to an animal (particularly, human), it is preferred that the solvent is an aqueous (or water-containing) solvent (in other words, water itself, or a mixture solvent containing water as a main component).

<Preferred combination of gel-forming mechanisms>

|  | Fast gel formation | Slow gel formation |
|---|---|---|
| (a) | Physical | Chemical |
| (b) | Chemical | Chemical |

(Embodiments of gel formation)

In the gel-forming composition according to the present invention, it is preferred to achieve a good balance between the "fast gel formation" and the "slow gel formation". It is preferred that the fast gel formation is predominantly governed by a gel-forming mechanism F, and the slow gel formation is predominantly governed by a gel-forming mechanism S, respectively. Preferred embodiments of these gel formations are as follows.

(Fast gel formation)

From a viewpoint that the gel-forming composition C (which shows the gel-forming mechanism F and the gel-forming mechanism S) according to the present invention exhibits a preferred short-term adhesion, the fast gel formation time (Tc) of the gel-forming composition C may preferably be 15 minutes or less, more preferably 10 minutes or less, particularly 5 minutes or less, in the following "fast gel formation" measurement.

Further, the gel formation time (Tf) for the "gel-forming mechanism F", when one component constituting the gel-forming composition C and corresponding to the gel-forming mechanism F is used singly, may preferably be 15 minutes or less, more preferably 10 minutes or less, particularly 5 minutes or less, in the following "fast gel formation" measurement.

In the present invention, it is preferred that the gel formation F is not substantially hindered in the gel-forming composition C. More specifically, the ratio (Tc/Tf) between the gel formation time (Tc) of the gel-forming composition C, and the gel formation time (Tf) of the "gel-forming mechanism F" when the corresponding component is used singly, may preferably be 3 or less, more preferably 2 or less, particularly 1.5 or less.

Either of the above-mentioned gel formation times Tf and Tc may preferably be measured under the condition for the "fast gel formation measurement". Herein, in a case where the gel-forming mechanism F is based on a physical gel formation, the condition for the "fast gel formation measurement" refers to a temperature which is in the side of gel formation region, wherein the difference between such a temperature and the sol-gel transition temperature is 10 °C (i.e., a temperature which is 10 °C higher or 10 °C lower than the sol-gel transition temperature).

On the other hand, in a case where the gel-forming mechanism F is based on a chemical gel formation, the condition for the "fast gel formation measurement" refers to the temperature at the site where the gel formation is to be effected. In other words, when the composition according to the present invention is used in the living body of an animal, the condition for the "fast gel formation measurement" refers to the body temperature of the animal (in the case of a human, about 38 °C). When the site to which the composition is to be applied is not specified, the condition for the "fast gel formation measurement" refers to room temperature (about 25 °C).

<Method of measuring fast gel formation>

(A case wherein gel-forming mechanism F is based on physical gel formation)

The gel-forming composition C according to the present invention is dissolved in a predetermined solvent (in the case of a hydrogel, distilled water) under stirring at a temperature which is about 10 °C lower than the sol-gel transition temperature of the above physical gel formation F, so as to prepare an aqueous solution of the gel-forming composition having a concentration of about 15 %.

Then, about 5 ml of the above-mentioned solution of the gel-forming composition C is poured into a glass test tube having a diameter of about 12 mmφ at the temperature which is about 10 °C lower than the sol-gel transition temperature, and thereafter the test tube is immersed in a constant-temperature water bath of about 38 °C. The period counted from the immersion in the constant-temperature water bath until the gel formation of the aqueous solution is measured. The gel formation at this time can be confirmed by observing a phenomenon that the aqueous solution does not show a fluidity, even when the test tube containing the aqueous solution of the gel-forming composition C is turned upside down.

Further, in a case where the gel-forming mechanism F is based on a chemical gel formation, the above-mentioned fast gel formation can be measured by obtaining a solution (about 15 % concentration) of the gel-forming composition C showing the gel-forming mechanism F and the gel-forming mechanism S; pouring the resultant solution into a glass test tube having a diameter of about 12 mmφ; and thereafter immersing the test tube in a constant-temperature water bath of about 38 °C.

(Measurement of slow gel formation)

From a viewpoint that the gel-forming composition C according to the present invention (showing the gel-forming mechanism F and the gel-forming mechanism S) shows a preferred long-term adhesion thereof, in the following "re-sol conversion-preventing" property in the following "slow gel formation" measurement, when the gel-forming composition C is subjected to the elapse of a slow gel formation time (Td) thereof.

<Method of measuring slow gel formation>

(A case wherein gel-forming mechanism F is based on physical gel formation)

The gel-forming composition C according to the present invention is dissolved in a predetermined solvent (in the case of a hydrogel, distilled water) under stirring at a temperature which is about 10 °C lower than the sol-gel transition temperature of the above physical gel formation F, so as to prepare an aqueous solution of the gel-forming composition having a concentration of about 15 %.

Then, about 10 ml of the above-mentioned solution of the gel-forming composition C is poured into a plastic Petri dish having an inside diameter of 80 mm$\phi$. Thereafter the Petri dish is placed in a heat-retaining oven of about 38 °C so as to convert the composition into a gel state, and then in the heat-retaining oven of 38 °C, about 40 ml of a pH 8-physiological saline solution of 38 °C is poured into the Petri dish containing the resultant gel. Under these circumstances, the Petri dish is left standing in the heat-retaining oven of 38 °C for about 1 hour (preferably, for about 5 hours, more preferably, about 24 hours) and the physiological saline solution is discarded. Even when the Petri dish containing the gel is placed in a refrigerator of about 6 °C and is left standing for at least one hour, the gel-forming composition C according to the present invention which has once been converted into a gel state is not changed into a sol state.

Further, in the case of the gel based on one component constituting the gel-forming composition C and corresponding the gel formation mechanism S, in the same "re-sol conversion-preventing" measurement as described above, the gel based on the gel formation mechanism S is not changed into a sol state, even when the gel is placed in a refrigerator of about 6 °C and is left standing for at least one hour.

In a case where the gel-forming mechanism F is based on a chemical gel formation, the same "re-sol conversion-preventing" property as described above can be recognized in the above-mentioned fast gel formation.

(Gel formation solely based on gel-forming mechanism S)

The gel formation time (Ts) when the gel-forming mechanism S is utilized alone, may preferably be one hour or more, more preferably five hours or more, particularly 24 hours or more. Herein, in a case where the gel-forming mechanism S is based on a chemical gel formation, the condition for the "slow gel formation measurement" refers to the temperature at the site where the gel formation is to be effected. In other words, when the gel is to be used in the interior of the body of an animal, the condition for the "slow gel formation measurement" refers to the body temperature of the animal (in the case of a human, about 38 °C). When the site to which the composition is to be applied is not specified, the condition for the "fast gel formation measurement" refers to room temperature (about 25 °C).

(Weight reduction in gel product in physiological saline solution)

From a viewpoint that the gel-forming composition C according to the present invention exhibits a good balance between the short-term adhesion and the long-term adhesion thereof, it is preferred that the gel-forming mechanism S providing a relatively slow gel formation is not substantially hindered by the presence of the crosslinking based on the fast gel-forming mechanism F.

More specifically, it is preferred that the gel-forming composition C (which shows a relatively fast gel-forming mechanism F and a relatively slow gel-forming mechanism S) and a gel-forming composition D for testing (which comprises a component corresponding to the gel-forming mechanism F and a component D1; and the component D1 is a component which has been obtained by selectively removing the "slow gel formation" ability substantially from the component corresponding to the gel-forming mechanism S) respectively show the following behaviors with respect to the weight reduction in a physiological saline solution of 38 °C. For example, the "component D1" can be obtained by selectively removing the component (e.g., catalyst such as enzyme and crosslinking agent) for providing a chemical crosslinking per se (or removing the crosslinking ability thereof) from the component corresponding to the gel-forming mechanism S.

The ratio (Cr/Dr) between the weight reduction of the gel-forming composition C and that of the gel-forming composition D may preferably be 0.4 or less, more preferably 0.2 or less (particularly 0.1 or less).

<Measurement of weight reduction in physiological saline solution>

About 5 g of a sample (a gel-forming composition C or D) to be measured is precisely weighed by means of a measuring device (such as electronic weighing instrument, trade name: EB-3200 D, mfd. by Shimazu Corporation; mass = C1 or D1 gram). Then, the sample is placed in a wire netting of 200 mesh (size: about $3 \times 3 \times 3$ cm), and the wire netting is suspended in a physiological saline solution having a salt concentration of 0.9 % filled in a beaker of 200 ml. The beaker is immersed into a constant-temperature water bath which has been set to 37 °C (temperature error: ± 1 °C or less), and is left standing for six hours while the contents in the beaker is stirred with a magnetic stirrer. After the standing, the sample in the wire netting is taken out and excessive physiological saline solution is removed therefrom with tissue paper, and then the mass of the sample (C2 or D2 gram) is measured. The resultant weight reduction ratio Cr or Dr is calculated by the following formula.

$$Cr = 100 \times (C1\text{-}C2)/C1$$

$$Dr = 100 \times (D1\text{-}D2)/D1$$

(Gel-forming material)

The "gel-forming mechanism F" corresponding to the fast gel formation and the "gel-forming S" corresponding to the slow gel formation may be the same mechanism or different mechanisms from each other, as long as they satisfy the conditions for the above-mentioned "fast gel formation" and "slow gel formation". In order to easily achieve a balance between the two kinds of gel formations having these different rates, the gel-forming mechanisms may preferably be different from each other. In this case, in view of the balance between the speed of the fast gel formation and the stability of the slow gel formation, it is preferred that the "gel-forming mechanism F" is a physical gel formation, and the "gel-forming mechanism S" is a chemical gel formation. In addition, it is preferred that the "gel-forming mechanism F" is a reversible gel formation, and the "gel-forming mechanism S" is an irreversible gel formation.

(Preferred embodiments of the gel-forming component F)

As described above, the gel-forming component F corresponding to the "gel-forming mechanism F" in the present invention may preferably be a material which is capable of causing a physical gel formation and/or reversible gel formation. The gel-forming component F may more preferably be one such that it can form a thermo-reversible hydrogel at an elevated temperature. In this case, such a thermo-gelling type thermo-reversible hydrogel formation may preferably be a physical gel wherein the crosslinking is constituted by hydrophobic bonding.
The gel-forming component F may preferably comprise a polymer such that an aqueous solution thereof has a sol-gel transition temperature, and it reversibly assumes a sol state at a temperature lower than the sol-gel transition temperature.
As specific examples of such a polymer, there have been known, e.g., polyalkylene-oxide block copolymer represented by block copolymers comprising polypropylene oxide portions and polyethylene oxide portions; etherified (or ether group-containing) celluloses such as methyl cellulose and hydroxypropyl cellulose; chitosan derivatives (K. R. Holme. et al. Macromolecules, <u>24</u>, 3828 (1991)), etc.
In addition, there has been developed a gel utilizing Pluronic F-127 (trade name, mfd. by BASF Wyandotte Chemical Co.) comprising a polypropylene oxide portion and polyethylene oxide portions bonded to the both terminals thereof.
It is known that a high-concentration aqueous solution of the above Pluronic F-127 is converted into a hydrogel at a temperature of not lower than about 20 °C and is converted into an aqueous solution at a temperature lower than this temperature. However, this material can assume a gel state only at a high concentration of not lower than about 20 wt. %. In addition, even when such a gel having a high concentration of not lower than about 20 wt.% is maintained at a temperature higher than the gel-forming temperature, the gel is dissolved when water is further added thereto. In addition, since the molecular weight of the Pluronic F-127 is relatively low, and it shows an extremely high osmotic pressure at a high concentration of not less than about 20 wt. %, and simultaneously the Pluronic F-127 may easily permeate the cell membranes, whereby the Pluronic F-127 can adversely affect cells and microorganisms.
On the other hand, in the case of an etherified cellulose represented by methyl cellulose, hydroxypropyl cellulose, etc., the sol-gel transition temperature thereof is as high as about 45 °C or higher (N. Sarkar, J. Appl. Polym. Science, <u>24</u>, 1073, (1979)). Accordingly, such an etherified cellulose is less liable to form a gel at body temperature (about 38 °C), and therefore it is difficult to use such a material for the above-mentioned purposes according to the present invention.
As described above, when a conventional polymer having a sol-gel transition temperature in an aqueous solution thereof, and reversibly assuming a sol state at a temperature lower than the above transition temperature is simply used, the following problems are posed:

(1) If the polymer is once converted into a gel state at a temperature higher than the sol-gel transition temperature, the resultant gel is dissolved when water is further added thereto;

(2) The polymer has a sol-gel transition temperature higher than the body temperature (in the neighborhood of 38 °C), and therefore the polymer assumes a sol state in the interior of a living body;

(3) It is necessary to increase the concentration of the polymer in an aqueous solution thereof to an extremely high value, in order to convert the polymer into a gel state; etc.

According to the present inventor's investigation, it has been found that a polymer which comprises a plurality of polymer chains having a cloud point, and a hydrophilic polymer chain block which has been bonded thereto; and an aqueous solution of which has a sol-gel transition temperature, and reversibly assumes a sol state at a temperature lower than the sol-gel transition temperature, may particularly preferably be used as a hydrogel-forming polymer constituting the above-mentioned gel-forming component F.

(Plural polymer chains having cloud point)

The polymer chain having a cloud point may preferably be a polymer which shows a negative solubility-temperature coefficient with respect to water. More specifically, such a polymer may preferably be one selected from the group of: polypropylene oxide, copolymers comprising propylene oxide and another alkylene oxide, poly N-substituted acrylamide derivatives, poly N-substituted methacrylamide derivatives, copolymers comprising an N-substituted acrylamide derivative and an N-substituted methacrylamide derivative, polyvinyl methyl ether, and partially-acetylated product of polyvinyl alcohol. It is preferred that the above polymer (polymer chain having a cloud point) has a cloud point of higher than 4 °C and not higher than 40 °C, in view of the provision of a polymer (compound comprising a plurality of polymer chains having a cloud point, and a hydrophilic polymer chain bonded thereto) to be used in the present invention having a sol-gel transition temperature of higher than 4 °C and not higher than 40 °C.

It is possible to measure the cloud point, e.g., by the following method. That is, an about 1 wt. %-aqueous solution of the above polymer (polymer chain having a cloud point) is cooled to be converted into a transparent homogeneous solution, and thereafter the temperature of the solution is gradually increased (temperature increasing rate: about 1 °C/min.), and the point at which the solution first shows a cloudy appearance is defined as the cloud point.

Specific examples of the poly N-substituted acrylamide derivatives and poly N-substituted methacrylamide derivatives to be usable in the present invention are described below.

Poly-N-acryloyl piperidine
Poly-N-n-propyl methacrylamide
Poly-N-isopropyl acrylamide
Poly-N,N-diethyl acrylamide
Poly-N-isopropyl methacrylamide
Poly-N-cyclopropyl acrylamide
Poly-N-acryloyl pyrrolidine
Poly-N,N-ethyl methyl acrylamide
Poly-N-cyclopropyl methacrylamide
Poly-N-ethyl acrylamide

The above polymer may be either a homopolymer or a copolymer comprising a monomer constituting the above polymer and "another monomer". The "another monomer" to be used for such a purpose may be a hydrophilic monomer, or a hydrophobic monomer. In general, when copolymerization with a hydrophilic monomer is conducted, the resultant cloud point may be increased. On the other hand, when copolymerization with a hydrophobic monomer is conducted, the resultant cloud point may be decreased. Accordingly, a polymer having a desired cloud point (e.g., a cloud point of higher than 4 °C and not higher than 40 °C) may be obtained by selecting such a monomer to be used for copolymerization.

Specific examples of the above hydrophilic monomer may include: N-vinyl pyrrolidone, vinyl pyridine, acrylamide, methacrylamide, N-methyl acrylamide, hydroxyethyl methacrylate, hydroxyethyl acrylate, hydroxymethyl methacrylate, hydroxymethyl acrylate, methacrylic acid and acrylic acid having an acidic group, and salts of these acids, vinyl sulfonic acid, styrenesulfonic acid, etc., and derivatives having a basic group such as N,N-dimethylaminoethyl methacrylate, N,N-diethylaminoethyl methacrylate, N,N-dimethylaminopropyl acrylamide, salts of these derivatives, etc. However, the hydrophilic monomer to be usable in the present invention is not restricted to these specific examples.

On the other hand, specific examples of the above hydrophobic monomer may include: acrylate derivatives and methacrylate derivatives such as ethyl acrylate, methyl methacrylate, and glycidyl methacrylate; N-substituted alkyl methacrylamide derivatives such as N-n-butyl methacrylamide; vinyl chloride, acrylonitrile, styrene, vinyl acetate, etc. However, the hydrophobic monomer to be usable in the present invention is not restricted to these specific examples.

(Hydrophilic polymer chain)

On the other hand, specific examples of the hydrophilic polymer chain to be combined with (or bonded to) the above-mentioned polymer chain having a cloud point, may include: collagen, gelatine, casein, albumin, methyl cellulose, dextran, polyethylene oxide, polyvinyl alcohol, poly N-vinyl pyrrolidone, polyvinyl pyridine, polyacrylamide, polymethacrylamide, poly N-methyl acrylamide, polyhydroxymethyl acrylate, polyacrylic acid, polymethacrylic acid, polyvinyl sulfonic acid, polystyrene sulfonic acid, and salts of these acids; poly N,N-dimethylaminoethyl methacrylate, poly N,N-diethylaminoethyl methacrylate, poly N,N-dimethylaminopropyl acrylamide, and salts of these, etc.

The process for combining the above polymer chain having a cloud point with the hydrophilic polymer chain is not particularly limited. For example, it is possible to conduct such a combination by introducing a polymerizable functional group (such as an acryloyl group) into either one of the above polymer chains, and copolymerizing with the resultant product a monomer capable of providing the other polymer chain. Alternatively, it is also possible to obtain a combination product of the above polymer chain having a cloud point with the hydrophilic polymer chain by copolymerizing a monomer capable of providing the polymer chain having a cloud point with a monomer capable of providing the hydrophilic polymer chain. In addition, the polymer chain having a cloud point and the hydrophilic polymer chain may also be combined or bonded with each other by preliminarily introducing reactive functional groups (such as hydroxyl group, amino group, carboxyl group, and isocyanate group) into both kinds of the polymer chains, and combining these polymer chains by using a chemical reaction. At this time, it is usual to introduce a plurality of reactive functional groups into the hydrophilic polymer chain.

Further, the polypropylene oxide having a cloud point and a hydrophilic polymer chain may be combined or bonded with each other by repetitively subjecting polypropylene oxide and a monomer constituting the above "other hydrophilic polymer chain" (such as ethylene oxide) to a stepwise or consecutive polymerization, to thereby obtain a polymer chain copolymer comprising polypropylene oxide and the "hydrophilic polymer chain" (such as polyethylene oxide) combined therewith. Such a block copolymer may also be obtained by introducing a polymerizable group (such as acryloyl group) into the terminal of polypropylene oxide, and then copolymerizing therewith a monomer constituting the hydrophilic polymer chain. Further, a gel-forming component F usable in the present invention may be obtained by introducing a functional group which is reactive in a bond-forming reaction with the terminal functional group of polypropylene oxide (such as hydroxyl group) into a hydrophilic polymer chain, and reacting the resultant hydrophilic polymer chain and the polypropylene oxide.

In addition, a gel-forming component F to be used in the present invention may be obtained by connecting a material such as one comprising polypropylene glycol and polyethylene glycol bonded to both terminals thereof (such as Pluronic F-127; trade name, mfd. by Asahi Denka Kogyo K.K.).

In an embodiment wherein a polymer chain having a cloud point is used, at a temperature lower than the cloud point, the gel-forming component F may completely be dissolved in water so as to assume a sol state, since the above-mentioned "polymer chain having a cloud point" present in the polymer molecule is water-soluble together with the hydrophilic polymer chain. However, when a solution of the above polymer is heated up to a temperature higher than the cloud point, the "polymer chain having a cloud point" present in the polymer molecule becomes hydrophobic so that separate molecules of the polymer are associated or aggregated with each other due to a hydrophobic interaction. On the other hand, the hydrophilic polymer chain is water-soluble even at this time (i.e., even when heated up to a temperature higher than the cloud point), and therefore, the gel-forming component F according to the present invention in water is formed into a hydrogel having a three-dimensional network structure wherein hydrophobic association portions between the polymer chains having a cloud point constitute crosslinking points. The temperature of the resultant hydrogel is again cooled to a temperature lower than the cloud point of the "polymer chain having a cloud point" present in the polymer molecule, the polymer chain having a cloud point becomes water-soluble and the above crosslinking points due to the hydrophobic association are released or liberated so that the hydrogel structure disappears, whereby the polymer according to the present invention is again formed into a complete aqueous solution.

In the above-described manner, the sol-gel transition in the polymer according to the present invention is, e. g., based on the reversible hydrophilic-hydrophobic conversion in the polymer chain having a cloud point present in the polymer molecule at the cloud point, and therefore the transition has a complete reversibility in accordance with a temperature change.

According to the present inventor's investigations and knowledge, even when an aqueous solution of the gel-forming component F according to the present invention is converted into a gel state at a temperature higher than the sol-gel transition temperature, and then the resultant gel was immersed in a large amount (about 0.1-100 times larger than the gel, by volume ratio), the gel is not dissolved. Such a property of the gel-forming component F according to the present invention may be provided, e.g., by using a polymer having a plurality (two or more) of polymer chains having a cloud point in one molecule as described above.

(Gel-forming component S)

When the gel-forming component F and the gel-forming component S constituting the gel-forming composition C according to the present invention have gel-forming mechanisms different from each other, as described above, the "gel-forming mechanism S" may preferably be a chemical gel formation. In addition, the "gel-forming mechanism S" may preferably be an irreversible gel formation. Further, as described above, the polymer component per se constituting the gel-forming component F, and the polymer component per se constituting the gel-forming component S may be the same or different from each other.

(Preferred embodiments of gel-forming component S)

As described above, the gel-forming component S in the present invention may preferably be a material which can cause a chemical gel formation and/or irreversible gel formation. The gel-forming mechanism of this the gel-forming component S is not particularly limited, as long as it satisfies the above-mentioned condition for the "slow gel formation". In an embodiment wherein the composition according to the present invention is used in the interior of the body of an animal (also including a human), the gel-forming component S may preferably comprise a gel-forming polymer which can be converted into a gel state even at a relatively low temperature (for example, not higher than 40 °C), and/or under a condition under which water is present.

In an embodiment wherein the composition according to the present invention is used in the interior of the body of an animal (particularly, a human), also in consideration of a problem of the short-term and long-term safety/toxicity thereof, the gel-forming component S may preferably comprise, at least, a gel-forming polymer, and a catalyst (preferably, enzyme) which is capable of converting the polymer into a gel state. In this case, when the above-mentioned issues including the gel-forming temperature, conditions relating to water, and safety/toxicity are considered comprehensively, it is preferred to use, as the gel-forming polymer, a biopolymer (or biological polymer) such as proteins, glycoproteins, and polypeptides, and/or derivatives thereof. It is preferred to use such a polymer substrate in combination with a catalyst (including an enzyme, as a biological catalyst) which is capable of converting the polymer substrate into a gel state. The gel-forming reaction of the gel-forming component S in this embodiment may preferably belong to a chemical reaction (particularly, an enzymatic reaction) or a chemical gel wherein the crosslinking is constituted by covalent bonding.

The combination of the polymer substrate and the catalyst (an enzyme, for example) which may preferably be used in the present invention is not particularly limited, as long as the combination satisfies the above-mentioned condition for the "slow gel formation". When safety issues are particularly taken seriously, it is particularly preferred to use the following combinations of polymer substrate-enzyme.

(Crosslinking reaction by transglutaminase)

Transglutaminase (TGase) is an enzyme which is widely distributed in higher animals, fishes and shellfish, and microorganisms, and catalyzes an acyl-transfer reaction of the $\gamma$-carboxyamide group of glutamine (Gln) residues contained in proteins and polypeptides. When the $\varepsilon$-amino group of lysine (Lys) residues contained in proteins and polypeptides acts as an acyl acceptor, an intramolecular and intermolecular $\varepsilon(\gamma$-glutamyl)lysine (hereinafter, abbreviated as "G-L") crosslinking bond (i.e., peptide bond) is formed, and protein molecules are crosslinked with each other so as to be converted into a gel state. As the most typical example, there is an enzyme, plasma transglutaminase (XIII factor) which forms the G-L crosskinking between fibrin molecules provided by the action of thrombin so as to remarkably improve the stability of blood clotting.

In addition, a large amount of epidermal transglutaminase is present in the keratinocytes constituting skin, and has a role of forming G-L crosslinking bonds between keratin molecules so as to form a tough and insoluble stratum corneum layer. In addition, in recent years, a large amount of transglutaminase (MTGase) originating from microorganisms has been produced inexpensively, and such an enzyme has industrially been used for gel formation in protein-containing foods.

As the polymer substrate which specifically reacts with TGase, or MTGase, there are present polypeptides, glycoproteins and proteins having the glutamine residue and the lysine residue. Examples of preferred polymer substrates to be used in the present invention may include: for example, collagen, gelatine, myosin, fibrinogen, fibrin, casein, keratin, $\gamma$-globulin, laminin, fibronectin, fibroblast growth factor (FGF), epithelial cell growth factor (EGF), vascular endothelial cell growth factor (VEGF), etc. Accordingly, one of preferred examples of the gel-forming component S according to the present invention includes a mixture of the above-mentioned polymer substrate, and TGase or MTGase.

On the other hand, in a case where the polymer chain (which is hydrophilic, in this case) as a component constituting the gel-forming component F comprises a protein such as collagen, gelatine, casein, and albumin having the glutamine residue and the lysine residue, the gel-forming component F forms a chemical gel with TGase or MTGase so as to play a role of the gel-forming component S according to the present invention. Accordingly, in such a case, both of the gel-forming component F (which comprises a polymer P) and the gel-forming component S (which comprises the polymer P and a gel-forming catalyst "Cat") include the common polymer P as a component constituting them.

(Crosslinking reaction by sulfhydryl oxidase)

Sulfhydryl oxidase (an S-S crosslinking enzyme) specifically reacts with a polymer substrate such as polypeptides, glycoproteins, and proteins having a thiol group, such as collagen, gelatine, myosin, fibrinogen, fibrin, casein, r keratin, albumin, y-globulin, laminin, a fibronectin, and insulin, so as to form a intramolecular or intermolecular disulfide bond, to thereby convert the substrate into a gel state. Accordingly, in such a case, the gel-forming component S includes a mixture of the above-mentioned polymer substrate and sulfhydryl oxidase.

On the other hand, when the hydrophilic chain as a component constituting the gel-forming component F according to the present invention comprises a protein having a thiol group such as collagen, gelatine, casein, and albumin, the gel-forming component F forms a chemical gel with an S-S crosslinking enzyme so as to play a role of the gel-forming component S according to the present invention. Accordingly, in such a case, both of the gel-forming component F (which comprises a polymer P) and the gel-forming component S (which comprises the polymer P and a gel-forming catalyst "Cat") include the common polymer P as a component constituting them.

(Crosslinking reaction by thrombin)

Thrombin which is a serine protease in the blood coagulation system converts fibrinogen into fibrin under physiological conditions (about 38 °C, in the neighborhood of pH 7), so as to insolubilize the fibrinogen, to thereby convert the fibrinogen into a gel state. Accordingly, in such a case, the gel-forming component S comprises a mixture of the above-mentioned polymer substrate and thrombin.

(Gel-forming reaction)

In an embodiment wherein the thermo-reversible hydrogel of thermo-gelling type as the gel-forming component F according to the present invention is a physical gel constituted by hydrophobic bonding, in general, the hydrophobic bonding force is influenced by a temperature change most greatly, while the hydrophobic bonding force is little influenced by a change in pH, or salt concentration. Accordingly, an aqueous solution of the above gel-forming polymer having a sol-gel transition temperature lower than the body temperature (about 38 °C) assumes a sol state at a temperature lower than the sol-gel transition temperature, and can easily be poured or injected into a desired site of a living body, so that the aqueous solution is promptly converted into a gel state at the body temperature (i.e., a temperature higher than the sol-gel transition temperature), and can stably be positioned at the above site. However, as described above, it is difficult to solely position the gel-forming component F with a stability for a long period at the site, for several reasons, including: that the crosslinking structure in the case of such a gel is constituted by hydrophobic bonding only effecting a weak bonding force, that the crosslinking structure is liable to become unstable due to a somewhat change in the temperature or concentration, that the mechanical strength thereof is low, etc.

On the other hand, in an embodiment wherein the gel-forming component S in the present invention is a mixture system which comprises a polymer substrate such as proteins, glycoproteins, polypeptides, and an enzyme which specifically reacts with the polymer substrate, and can form a chemical gel having a crosslinking structure constituted by covalent bond, the gel-forming reaction of the above chemical gel is an enzymatic reaction. Accordingly, such a reaction proceeds under conditions such that the concentration, pH, kind and concentration of salt are within the physiological conditions (e.g., temperature is about 38 °C, pH is in a neutral region, and the kind and concentration of salt are those comparable to those in physiological saline solution or Ringer's solution) so as to form a gel.

Accordingly, when the concentration, pH, kind and concentration of salt are not within the physiological conditions, the aqueous solution of the gel-forming component S assumes a sol state, because the gel-forming reaction does not proceed under the non-physiological conditions. Therefore, the aqueous solution of the gel-forming component S can easily be injected into a living body at a desired site thereof, and the gel-forming reaction of the gel-forming component S proceeds, as various conditions such as concentration, pH, kind and concentration of salt are gradually changed to the physiological conditions at the above site.

The above gel-forming reaction rate is much slower than the gel-forming reaction rate of the "fast" gel-forming component F, and therefore it is very difficult to position the gel-forming component S solely with stability at a predetermined site in the living body. When an aqueous solution of the composition (II) is injected into a living body under the physiological conditions in order to promote the gel-forming reaction of the gel-forming component S, the gel-forming reaction also proceeds during the preparation of the aqueous solution, or in the course of the injection thereof into the living body. As a result, it is difficult to inject the composition into a desired site in the living body. In other words, it is very difficult to most favorably control the rate of the gel-forming reaction, when only the gel-forming component S is used. However, the gel based on the gel-forming component S can be positioned at a desired site stably for a long period, because the crosslinking structure of the gel-forming component S is constituted by covalent bonding, and has an excellent stability and mechanical strength to a change in the external factor such as temperature and concentration.

In the present invention, in order to solve the above-mentioned problems encountered in the single use of the gel-forming reaction of the gel-forming component F or the gel-forming component S, the gel-forming composition C is constituted by combining the gel-forming component F and the gel-forming component S. That is, in an embodiment wherein the gel-forming composition C according to the present invention is applied to a living body, for example, it is possible that an aqueous solution of the gel-forming composition C (in a sol state) is prepared under non-physiological conditions of temperature, pH, kind and concentration of salt, at temperature lower than the sol-gel transition temperature of the gel-forming component F; the resultant solution is injected into a living body so that the temperature of the gel-forming composition C is increased to a temperature higher than the sol-gel transition temperature under the influence of the body temperature, the gel-forming composition C is converted into a gel state because the gel-forming component F is first converted into a gel state. Then, as the temperature, pH, kind and concentration of salt in the gel are changed to near the physiological conditions, an enzymatic reaction of the gel-forming component S proceeds so as to form a chemical gel of the gel-forming component S, and there is formed a stabilized gel based on the gel-forming composition C according to the present invention.

When a crosslinking reaction by transglutaminase, sulfhydryl oxidase is utilized as a chemical gel-forming reaction, it is preferred that an aqueous solution of the gel-forming composition C according to the present invention (in a sol state) is prepared at a temperature which is much lower than the body temperature (about 38 °C) and is lower than the sol-gel transition temperature of the gel-forming component F, and the resultant solution is administered into the interior of a living body. When the temperature of the composition is changed to near the body temperature in the living body, an enzymatic reaction proceeds so as to contribute to the stabilization of the gel-forming composition C according to the present invention.

When a crosslinking reaction by thrombin is utilized as a chemical gel-forming reaction, it is possible to suppress the progress of the crosslinking reaction of fibrinogen with thrombin by decreasing the temperature of the composition to a temperature which is sufficiently lower than the body temperature, or by adjusting the pH to a value lower than 5 or higher than 10, at the time of the preparation of the composition or injection thereof into a living body. When the temperature of the composition or pH is gradually changed to the physiological conditions in the living body, the fibrinogen is changed to crosslinked fibrin so as to contribute to the stabilization of the gel-forming composition C according to the present invention.

(Other components)

As described above, the gel-forming composition according to the present invention comprises at least a polymer component, and a gel-forming agent for converting the polymer component into a gel state, but the gel-forming composition may also comprise another component as desired. The kind, number, content, etc., of such a component are not particularly limited, as long as it does not substantially hinder the balance between the fast gel formation F and a relatively slow gel formation S of the above composition.

Examples of such "other component" may include pharmaceuticals (or drugs), physiologically active substances and/or cells.

Preferred examples of the above pharmaceutical may include anti-cancer agents, antimicrobial agents, anticoagulant agents, etc. When a pharmaceutical is contained in the gel-forming composition according to the present invention in this way, it is possible to obtain the effect corresponding to the action of the pharmaceutical (e.g., anticancer activity, antibacterial activity, anticoagulant activity, etc.).

Preferred examples of the above physiologically active substance may include, e.g., FGF, EGF, VEGF, etc. When such a physiologically active substance is included in the gel-forming composition according to the present invention in this way, it is possible to obtain an effect of a healing-promoting activity, etc.

Preferred examples of the above cell may include marrow cell, fibroblast, vascular endothelial cell, etc. When such a cell is included in the gel-forming composition according to the present invention in this way, it is possible to obtain an effect of a healing-promoting activity, etc.

Hereinbelow, the present invention will be described in more detail with reference to Examples.

Examples

Example 1

10 g of a polypropylene oxide-polyethylene oxide copolymer (average polymerization degree of propylene oxide/ethylene oxide: about 60/180, Pluronic F-127 mfd. by Asahi Denka Kogyo K.K.) was dissolved in 30 ml of dry chloroform, and 0.13 g of hexamethylene diisocyanate was added thereto in the presence of phosphorous pentaoxide, and the resultant mixture was subjected to reaction at the boiling point under reflux for six hours. The solvent was distilled off under reduced pressure from the reaction mixture, and then the residue was dissolved in water, and the resultant mixture was subjected to ultrafiltration having a molecular cutoff of 30000, so as to separate a high-molecular weight polymer and a low-molecular weight polymer. The thus obtained aqueous solutions were frozen to thereby provide a high-molecular weight F-127 polymer and a low-molecular weight F-127 polymer.

The high-molecular weight F-127 polymer (TGP-1) which had been obtained in the above procedure was dissolved in distilled water at 4 °C so as to provide a concentration of 8 mass %. As the resultant aqueous solution is slowly warmed, the viscosity thereof gradually increased at a temperature of 21 °C or higher, and was solidified at about 27 °C so as to provide a hydrogel. When the hydrogel was cooled, it returned to an aqueous solution at 21 °C. This change was observed repeatedly and reversibly. On the other hand, when the low-molecular weight F-127 polymer which had been obtained in the above procedure was dissolved in distilled water so as to provide a concentration of 8 mass %. However, the resultant aqueous solution was not converted into a gel at all, even when the aqueous solution was heated to 60 °C or higher.

Separately, a 10 %-aqueous solution of an alkali-treated gelatine obtained from bone of adult bovine (mfd. by Nitta Gelatine Co.) was prepared, and in the thus obtained aqueous solution, the high-molecular weight F-127 polymer (TGP-1) which had been obtained in the above procedure was dissolved under stirring at about 10 °C which was lower than the sol-gel transition temperature of TGP-1, so as to provide a concentration of about 10 %. Then, transglutaminase (MTGase) was dissolved in the resultant aqueous solution, at about 10 °C under stirring so as to provide a concentration of about 20 unit/ml in terms of the enzyme concentration, to thereby obtain an aqueous solution (I) of the gel-forming composition C according to the present invention.

About 5 ml of the thus obtained aqueous solution (I) of the gel-forming composition C was poured into a glass test tube having an inside diameter of about 12 mmϕ at about 10 °C, and the test tube was immersed in a constant-temperature water bath of about 38 °C. At this time, the aqueous solution (I) was converted into a gel state promptly (i.e., when the temperature of the aqueous solution was increased to a temperature higher than the sol-gel transition temperature of TGP-1). The gel formation of the aqueous solution was confirmed on the basis of a phenomenon that the resultant gel did not exhibit a fluidity even when the test tube containing the above gel was turned upside down. After about 30 minutes from the point where the test tube containing the gel had been immersed in a constant-temperature water bath of about 38 °C, when the test tube containing the gelled aqueous solution (I) was immersed in a constant-temperature water bath of about 10 °C, the aqueous solution (I) was converted into a sol state promptly (i. e., when the temperature of the aqueous solution was decreased to a temperature lower than the sol-gel transition temperature of TGP-1).

On the contrary, after about 6 hours from the point where the test tube containing the gel had been immersed in a constant-temperature water bath of about 38 °C, when the test tube containing the gelled aqueous solution (I) was immersed in a constant-temperature water bath of about 10 °C, the conversion of the aqueous solution (I) into a sol state was not observed.

As described above, it was found that the aqueous solution of the gel-forming composition C(I) according to the present invention assumed a sol state (and was in a solution state) at a temperature lower than the sol-gel transition temperature of the gel-forming component F (TGP-1), the aqueous solution could easily be administered into a living body; and that the TGP-1 was promptly converted into a gel state after the administration thereof into a living body, so that the resultant gel could be positioned in the administration site. In addition, in the case of the gel-forming composition C (I), the gel-forming component S gradually formed a gel by the enzymatic reaction of MTGase at body temperature (about 38 °C), whereby the stability of the gel-forming composition C(I) according to the present invention was remarkably improved.

Example 2

In the same manner as in Example 1, a 10 %-aqueous solution of an alkali-treated gelatine was prepared, and in the thus obtained aqueous solution, the high-molecular weight F-127 polymer (TGP-1) which had been synthesized in Example 1 was dissolved under stirring at about 10 °C which was lower than the sol-gel transition temperature of TGP-1, so as to provide a concentration of about 10 %. Then, sulfhydryl oxidase was dissolved in the resultant aqueous solution at about 10 °C under stirring so as to provide a concentration of about 10 unit/ml in terms of the enzyme concentration, to thereby obtain an aqueous solution (II) of the gel-forming composition C according to the present invention.

About 5 ml of the thus obtained aqueous solution (II) of the gel-forming composition C was poured into a glass test tube having an inside diameter of about 12 mm$\phi$ at about 10 °C, and the test tube was immersed in a constant-temperature water bath of about 38 °C. At this time, the aqueous solution (II) was converted into a gel state promptly (i.e., when the temperature of the aqueous solution was increased to a temperature higher than the sol-gel transition temperature of TGP-1). The gel formation of the aqueous solution was confirmed on the basis of a phenomenon that the resultant gel did not exhibit a fluidity even when the test tube containing the above gel was turned upside down. After about one hour or less from the point where the test tube containing the gel had been immersed in a constant-temperature water bath of about 38 °C, when the test tube containing the gelled aqueous solution (II) was immersed in a constant-temperature water bath of about 10 °C, the aqueous solution (II) was converted into a sol state promptly (i.e., when the temperature of the aqueous solution was decreased to a temperature lower than the sol-gel transition temperature of TGP-1).

On the contrary, after about 10 hours from the point where the test tube containing the gel had been immersed in a constant-temperature water bath of about 38 °C, when the test tube containing the gelled aqueous solution (II) was immersed in a constant-temperature water bath of about 10 °C, the conversion of the aqueous solution (II) into a sol state was not observed.

As described above, it was found that the aqueous solution of the gel-forming composition C(II) according to the present invention assumed a sol state (and was in a solution state) at a temperature lower than the sol-gel transition temperature of the gel-forming component F (TGP-1), the aqueous solution could easily be administered into a living body; and that the TGP-1 was promptly converted into a gel state after the administration thereof into a living body, so that the resultant gel could be positioned in the administration site. In addition, in the case of the gel-forming composition C(II), the gel-forming component S gradually formed a gel by the enzymatic reaction of sulfhydryl oxidase at body temperature (about 38 °C), whereby the stability of the gel-forming composition C(II) according to the present invention was remarkably improved.

Example 3

Bovine fibrinogen (mfd. by Wako Pure Chemical Co.) was dissolved in a physiological saline solution which had been adjusted to pH 5 so as to provide a concentration of about 20 g/l. In the thus obtained aqueous solution, the high-molecular weight F-127 polymer (TGP-1) which had been synthesized in Example 1 was dissolved under stirring at about 10 °C, which was lower than the sol-gel transition temperature of TGP-1, so as to provide a concentration of about 10 %. Then, bovine thrombin was dissolved in the resultant aqueous solution at 10 °C so as to provide a concentration of about 1.0 unit/ml, to thereby obtain an aqueous solution (III) of the gel-forming composition C according to the present invention.

About 10 ml of the thus obtained aqueous solution (III) of the gel-forming composition C was poured into a plastic Petri dish having an inside diameter of 80 mm$\phi$ at about 10 °C, and the Petri dish was placed in a heat-retaining oven of about 38 °C. At this time, the aqueous solution (III) was converted into a gel state promptly (i.e., when the temperature of the aqueous solution was increased to a temperature higher than the sol-gel transition temperature of TGP-1). When the Petri dish containing the aqueous solution (III) which had been converted into a gel state in the heat-retaining oven of about 38 °C, was placed in a refrigerator of about 6 °C, the aqueous solution (III) was converted into a sol state promptly.

On the contrary, about 40 ml of physiological saline of pH 8 was added onto the aqueous solution (III) in the Petri dish which had been converted into a gel state in the constant-temperature oven of about 38 °C, and the Petri dish was left standing for one hour in the constant-temperature oven of 38 °C. Even when the above physiological saline was discarded, and then the Petri dish was placed in a refrigerator at about 6 °C, the conversion of the aqueous solution (III) into a sol state was not observed.

As described above, it was found that the aqueous solution of the gel-forming composition C(III) according to the present invention assumed a sol state at a temperature lower than the sol-gel transition temperature of the gel-forming component F (TGP-1), and at about pH 5 of the aqueous solution, the aqueous solution could easily be administered into a living body; and that the TGP-1 was promptly converted into a gel state after the administration thereof into a living body, so that the resultant gel could be positioned in the administration site. In addition, in the case of the gel-forming composition C(III), when the temperature of the gelled product was changed near to body temperature (about 38 °C) and the pH of the gelled product was changed near to the pH value of a living body, the thrombin converted the fibrinogen into fibrin, whereby the stability of the gel-forming composition C(III) according to the present invention was remarkably improved.

Industrial Applicability

As described hereinabove, according to the present invention, there is provided a composition having a gel-forming property, comprising, at least, a polymer component, and a gel-forming agent for converting the polymer component into a gel state; the gel-forming composition showing at least two kinds of the gel formations including a relatively fast gel formation and a relatively slow gel formation.

The present invention also provides a composition having a gel-forming property, comprising, at least, a polymer component, and a gel-forming agent for converting the polymer component into a gel state; the gel-forming composition showing at least two kinds of the gel formations including a relatively fast gel formation F and a relatively slow gel formation S, wherein the gel formation F and the gel formation S are not substantially hindered with each other.

The present invention further provides a composition having a gel-forming property, comprising, at least, a polymer component, and a gel-forming agent for converting the polymer component into a gel state; the gel-forming composition showing at least two kinds of the gel formations including a relatively fast gel formation F and a relatively slow gel formation S, wherein the gel formation F and the gel formation S have mutually different gel-forming mechanisms from each other.

The present invention further provides a composition having a gel-forming property, comprising, at least, a polymer component, and a gel-forming agent for converting the polymer component into a gel state; the gel-forming composition showing at least two kinds of the gel formations including a reversible and relatively fast gel formation F, and an irreversible and relatively slow gel formation S.

The gel-forming composition according to the present invention can easily realize a good balance between the short-term adhesion thereof (such as the adhesion of an instant adhesive) and the long-term adhesion thereof (such as delayed adhesion having durability). Accordingly, the gel-forming composition according to the present invention can be applied to fields wherein such a balance is demanded (e.g., adhesion in a fluid or liquid, repair of a fluid passage pipe) without particular limitation.

When the gel-forming composition according to the present invention is applied to a medical field, for example, it is possible that the gel-forming composition is injected or poured into a living body in the form of an aqueous solution (sol) state so as to be converted into a gel state quickly, whereby the resultant gel is positioned or indwelled at the injection site. In this case, the gel-forming composition is gradually gelled in the living body, whereby the mechanical strength of the composition can be improved, and the stability thereof can remarkably be improved. Accordingly, in this case, this composition is widely usable as a so-called "instant adhesive" also having a stability to be used in a living body (such as intravascular embolus-forming agent, hemostatic agent, in-vivo adhesive, adhesion-preventing agent, wound-covering agent, and base material for drug delivery).

## Claims

1. A composition having a gel-forming property, comprising, at least, a polymer component, and a gel-forming agent for converting the polymer component into a gel state; the gel-forming composition showing at least two kinds of the gel formations including a relatively fast gel formation and a relatively slow gel formation.

2. A composition having a gel-forming property, comprising, at least, a polymer component, and a gel-forming agent for converting the polymer component into a gel state; the gel-forming composition showing at least two kinds of the gel formations including a relatively fast gel formation F and a relatively slow gel formation S, wherein the gel formation F and the gel formation S are not substantially hindered by each other.

3. A composition having a gel-forming property, comprising, at least, a polymer component, and a gel-forming agent for converting the polymer component into a gel state; the gel-forming composition showing at least two kinds of the gel formations including a relatively fast gel formation F and a relatively slow gel formation S, wherein the gel formation F and the gel formation S have mutually different gel-forming mechanisms from each other.

4. A composition having a gel-forming property, comprising, at least, a polymer component, and a gel-forming agent for converting the polymer component into a gel state; the gel-forming composition showing at least two kinds of the gel formations including a reversible and relatively fast gel formation F, and an irreversible and relatively slow gel formation S.

5. A composition having a gel-forming property according to any of claims 1-4, wherein either one of the two kinds of gel formations F and S is based on a physical gel formation, and the other is based on a chemical gel formation.

**6.** A composition having a gel-forming property according to claim 5, wherein the gel formations F is based on a physical gel formation, and the gel formation S is based on a chemical gel formation.

**7.** A composition having a gel-forming property according to any of claims 1-6, wherein both of the components corresponding to the gel formation F and the gel formation S are in the form of a polymer solution or polymer dispersion.

**8.** A composition having a gel-forming property according to any of claims 1-7, wherein the component corresponding to the gel formation F is an aqueous solution of a polymer component (I) which has a sol-gel transition temperature, assumes a sol state at a lower temperature, and assumes a gel state at a higher temperature; and the component corresponding to the gel formation S is an aqueous solution of a polymer component (II) which forms a gel under physiological conditions (37-39 °C, pH 7.3 to 7.6).

**9.** A composition having a gel-forming property according to claim 8, wherein the polymer component (I) for providing the gel formation F comprises a polymer chain having a cloud point, and a hydrophilic polymer chain.

**10.** A composition having a gel-forming property according to claim 9, wherein the polymer component (I) for providing the gel formation F comprises a plurality of polymer chains having a cloud point, and a hydrophilic polymer chain.

**11.** A composition having a gel-forming property according to any of claims 8-10, wherein the polymer component (I) for providing the gel formation F has a sol-gel transition temperature of not lower than 4 and not higher than 40 °C.

**12.** A composition having a gel-forming property according to any of claims 8-11, wherein an aqueous solution (sol) of the polymer component (I) for providing the gel formation F at a temperature lower than the sol-gel transition temperature is rapidly converted into a gel state at about 38 °C.

**13.** A composition having a gel-forming property according to any of claims 8-12, wherein the polymer component (II) for providing the gel formation S comprises at least a polymer substrate, and a catalyst specifically reacting with the polymer substrate so as to convert the polymer substrate into a gel state.

**14.** A composition having a gel-forming property according to any of claims 8-13, wherein the polymer component (II) for providing the gel formation S comprises a protein, a glycoprotein and/or a polypeptide.

**15.** A composition having a gel-forming property according to claim 13 or 14, wherein the polymer component (II) for providing the gel formation S comprises an enzyme as the catalyst.

**16.** A composition having a gel-forming property according to claim 15, wherein the polymer substrate for providing the gel formation S has a glutamine residue and a lysine residue, and the enzyme comprises transglutaminase.

**17.** A composition having a gel-forming property according to claim 15, wherein the polymer substrate for providing the gel formation S has a thiol residue, and the enzyme comprises sulfhydryl oxidase (an S-S crosslinking enzyme).

**18.** A composition having a gel-forming property according to claim 15, wherein the polymer substrate for providing the gel formation S comprises a coagulation system protein, and the enzyme comprises serine protease.

**19.** A composition having a gel-forming property according to claim 15, wherein the polymer substrate for providing the gel formation S comprises fibrinogen, and the enzyme comprises thrombin.

**20.** A composition having a gel-forming property according to any of claims 8-19, wherein the polymer component (II) for providing the gel formation S is not formed into a gel state under a non-physiological condition, but is formed into a gel state under a physiological condition.

**21.** A composition having a gel-forming property according to claim 20, wherein the composition is gradually formed into a gel state under a physiological condition, when an aqueous solution of the polymer component (II) for providing the gel formation S is injected into a living body under a non-physiological condition.

**22.** A composition having a gel-forming property according to any of claims 8-21, wherein when an aqueous solution (sol) of the gel-forming composition is injected into a living body at a temperature lower than the sol-gel transition temperature of the polymer component (I) for providing the gel formation F, and under a non-physiological condition, the polymer component (I) is rapidly converted into a gel state, and the polymer component (II) for providing the gel formation S is gradually formed into a gel state under a physiological condition.

23. A composition having a gel-forming property according to any of claims 1-22, which further comprises a pharmaceutical, a physiologically active substance, and/or cell.

## INTERNATIONAL SEARCH REPORT

| International application No. |
| --- |
| PCT/JP01/04277 |

A. CLASSIFICATION OF SUBJECT MATTER
Int.Cl⁷ C08L101/00, C08K5/00, A61L31/04, A61L24/06, A61K9/08, A61P17/02, A61P7/04, A61P43/00

According to International Patent Classification (IPC) or to both national classification and IPC

B. FIELDS SEARCHED

Minimum documentation searched (classification system followed by classification symbols)
Int.Cl⁷ C08L1/00-101/16, C08K3/00-13/08, A61L31/04, A61L24/06, A61K9/08, A61P17/02, A61P7/04, A61P43/00

Documentation searched other than minimum documentation to the extent that such documents are included in the fields searched

Electronic data base consulted during the international search (name of data base and, where practicable, search terms used)
WPI/L

C. DOCUMENTS CONSIDERED TO BE RELEVANT

| Category* | Citation of document, with indication, where appropriate, of the relevant passages | Relevant to claim No. |
| --- | --- | --- |
| X | JP 11-169703 A (M & M Kenkyusho K.K.), 29 June, 1999 (29.06.99), Claims; working example (Family: none) | 1-3,7-12,14,23 |
| X | JP 63-230041 A (Meiji Milk Products Co., Ltd.), 26 September, 1988 (26.09.88), Claims; working example (Family: none) | 1-3 |
| A | JP 5-255119 A (W R Grace & Co.), 05 October, 1993 (05.10.93), Claims (Family: none) | 1-23 |
| A | JP 57-164064 A (Toray Industries, Inc.), 08 October, 1982 (08.10.82), Claims (Family: none) | 1-23 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 09 August, 2001 (09.08.01) | Date of mailing of the international search report 21 August, 2001 (21.08.01) |
| --- | --- |
| Name and mailing address of the ISA/ Japanese Patent Office | Authorized officer |
| Facsimile No. | Telephone No. |

Form PCT/ISA/210 (second sheet) (July 1992)